# NOUVEAU FASCICULE DE BREVET EUROPEEN

(11) **EP 0 557 512 B2**
(45) Date de publication et mention de la décision concernant l'opposition: **08.04.2009**
(45) Mention de la délivrance du brevet: 06.12.1995
(21) Numéro de dépôt: 92920525.0
(22) Date de dépôt: 15.09.1992
(51) Int. Cl.: A61K 8/892, A61K 8/58, A61K 8/891, A61K 8/893, A61K 8/899, A61K 9/00, A61K 47/32, A61K 47/34, A61Q 5/00, A61Q 19/00, A61K 8/897

(54) **UTILISATION EN COSMETIQUE OU EN APPLICATION TOPIQUE D'UNE DISPERSION AQUEUSE A BASE D'ORGANOPOLYSILOXANES ET D'UN COPOLYMERE RETICULE D'ACRYLAMIDE/ACID 2-ACRYLAMIDO 2-METHYLPROPANE SULFONIQUE NEUTRALISE**
VERWENDUNG DER KOSMETIKA UND TOPISCHE ANWENDUNG EINER WÄSSRIGEN DISPERSION EINER ORGANOPOLYSILOXANE UND EINER VERNETZTE COPOLYMER
UTILIZATION IN COSMETIC OR TOPICAL APPLICATIONS OF AN AQUEOUS DISPERSION BASED ON ORGANOPOLYSILOXANES AND A CROSSLINKED COPOLYMER OF ACRYLAMIDE/NEUTRALIZED 2-ACRYLAMIDO 2-METHYLPROPANE SULFONIC ACID

(30) Priorité: 17.09.1991 FR 9111439
(43) Date de publication de la demande: 01.09.1993
(73) Titulaire: L'ORÉAL, 75008 Paris (FR)
(72) Inventeur: DUBIEF, Claude, F-78150 Le Chesnay (FR); CAUWET, Danièle, F-75011 Paris (FR)
(74) Mandataire: Casalonga, Axel
(86) Numéro de dépôt international: PCT/FR1992/000866
(87) Numéro de publication internationale: WO 1993/005762

(56) Documents cités:
- EP-A1- 0 424 260
- EP-A1- 0 424 260
- EP-A2- 0 186 361
- EP-A2- 0 359 349
- EP-A2- 0 466 184
- EP-A2- 0 466 184
- WO-A1-92/21316
- FR- - 9 106 657
- US- - 4 859 458
- US- - 5 034 218

## Description

L'invention concerne l'utilisation pour le traitement cosmétique des cheveux, d'une dispersion aqueuse à base d'organopolysiloxanes et d'un copolymère réticulé d'acrylamide/acide 2-acrylamido 2-méthyl propane sulfonique neutralisé.

On utilise déjà des huiles siliconées en cosmétique comme lubrifiant dans les compositions de traitement des cheveux et de la peau. Il s'agit principalement de polydiméthylsiloxanes.

Afin d'apporter la douceur aux cheveux ou à la peau, ou encore de faciliter le démêlage des cheveux, on utilise depuis longtemps des polymères ou des tensio-actifs cationiques. Les composés cationiques présentent l'inconvénient, après applications répétées, d'alourdir la chevelure en lui donnant un aspect poisseux ou de produire un effet collant sur la peau.

On connait dans l'état de la technique des dispersions aqueuses contenant l'association d'un organopolysiloxane et d'un copolymère acrylate d'ammonium/acrylamide réticulé telles que celles décrites dans le document EP 0 424 260.

La demanderesse a découvert, d'une manière surprenante, que l'utilisation d'une dispersion aqueuse à base d'organopolysiloxanes et d'un copolymère réticulé d'acrylamide/acide 2-acrylamido 2-méthyl propane sulfonique neutralisé pour le traitement des cheveux, permet d'obtenir des cheveux brillants, soyeux, légers, dont les propriétés de démêlage et de douceur sont sensiblement améliorées.

Les dispersions aqueuses utilisées pour le traitement cosmétique des cheveux, selon la présente invention, se répartissent beaucoup plus facilement sur les cheveux que les compositions de l'art antérieur à base de composés cationiques.

La Demanderesse a découvert également que les compositions cosmétiques sous forme de dispersion aqueuse, selon la présente invention, étaient remarquablement stables, que leurs propriétés cosmétiques se conservaient même après plusieurs applications successives et plus particulièrement en application non rincée sur les cheveux.

Un objet de l'invention est donc constitué par l'utilisation dans le traitement cosmétique des cheveux, d'une dispersion aqueuse contenant au moins un organopolysiloxane et d'un copolymère réticulé d'acrylamide/acide 2-acrylamido 2-méthylpropane sulfonique neutralisé.

Un autre objet de l'invention concerne des compositions cosmétiques pour le traitement des cheveux, sous forme de dispersions aqueuses.

Un autre objet de l'invention concerne des procédés de traitement cosmétique des cheveux, mettant en oeuvre ces compositions, selon l'application désirée.

D'autres objets de l'invention apparaîtront à la lumière de la description et des exemples qui suivent.

La présente invention a pour objet principal l'utilisation pour le traitement cosmétique des cheveux, d'une dispersion aqueuse, caractérisée par le fait que celle-ci contient au moins dans un milieu aqueux cosmétiquement ou physiologiquement acceptable, un organopolysiloxane et un copolymère réticulé d'acrylamide/acide 2-acrylamido 2-méthylpropane sulfonique neutralisé; sous réserve que l'organopolysiloxane ne soit pas choisi parmi les polydiméthylsiloxanes linéaires de viscosité inférieure à 10⁻¹ m²/s.

Les organopolysiloxanes utilisés dans les dispersions selon la présente invention, sont des huiles d'organopolysiloxanes ou des solutions organiques de gomme ou de résine d'organosiloxanes.

Parmi les organosiloxanes utilisés conformément à la présente invention, on peut citer à titre non limitatif :

### 1. Les silicones volatiles.

Celles-ci possèdent un point d'ébullition compris entre 60 ° C et 260°C. Parmi ce type de silicones, on cite :
(i) les silicones cycliques de 3 à 7 atomes de silicium et de préférence 4 à 5. Il s'agit, par exemple, de l'octaméthylcyclotétrasiloxane vendu sous le nom de VOLATILE SILICONE 7207 par UNION CARBIDE ou SILBIONE 70045 V2 par RHONE POULENC, le décaméthylcyclopentasiloxane vendu sous le nom de VOLATILE SILICONE 7158 par UNION CARBIDE, SILBIONE 70045 V5 par RHONE POULENC, ainsi que leurs mélanges.
   On cite également les cyclocopolymères du type diméthylsiloxane/méthylalkylsiloxane, tel que la SILICONE VOLATILE FZ 3109 vendue par la Société UNION CARBIDE, qui est un cyclocopolymère diméthylsiloxane/méthyloctylsiloxane;
(ii) les silicones volatiles linéaires ayant 2 à 9 atomes de silicium et possédant une viscosité inférieure ou égale à 5.10⁻⁶ m²/s à 25°C. Il s'agit, par exemple, de l'hexaméthyldisiloxane vendu sous la dénomination SILBIONE 70 041 V 0,65 par la Société RHONE POULENC. Ce type de produit est décrit dans l'article de TODD & BYERS "Volatile silicone fluids for cosmetics", Cosmetics and Toiletries, Vol. 91, Jan 76, p. 27-32.

### II. Les silicones non volatiles.

Elles sont constituées principalement par les polyalkylsiloxanes, les polyarylsiloxanes, les polyalkylarylsiloxanes, les gommes et résines de silicone et les polysiloxanes organomodifiés, ainsi que leurs mélanges.

Parmi les polyalkylsiloxanes, on peut citer principalement les polydiméthylsiloxanes linéaires de viscosité supérieure à 10⁻¹ m²/s : soit,
- à groupements terminaux triméthylsilyle, comme par exemple, et à titre non limitatif, les huiles SILBIONE de la série 70047 commercialisées par RHONE POULENC : l'huile 47 V 500.000 de RHONE POULENC ou certaines Viscasil de la GENERAL ELECTRIC, ou
- à groupements terminaux trihydroxysilyle, telles que les huiles de la série 48 V de RHONE POULENC.

Dans cette classe de polyalkylsiloxanes, on peut également mentionner les polyalkylsiloxanes vendus par la Société GOLDSCHMIDT sous les dénominations ABILWAX 9800 et ABILWAX 9801, qui sont des polyalkyl(C₁ -C₂₀)siloxanes.

Parmi les polyalkylarylsiloxanes, on peut citer les polydiméthylphénylsiloxanes, les polydiméthyldiphénylsiloxanes linéaires et/ou ramifiés, de viscosité 10⁻⁵ à 5.10⁻² m²/s à 25°C, tels que, par exemple :
- l'huile RHODORSIL 763 de RHONE POULENC,
- les huiles SILBIONE de la série 70641 de RHONE POULENC, telles que les huiles SILBIONE 70641 V 30 et 70641 V 200 de RHONE POULENC,
- le produit DC 556 Cosmetic Grad Fluid de DOW CORNING,
- les silicones des séries PK de BAYER, telles que la PK20,
- les silicones des séries PN, PH de BAYER, comme les PN 1000 et PH 1000,
- certaines huiles des séries SF de GENERAL ELECTRIC, telles que les SF 1250, SF 1265, SF 1154, SF1023.

Les gommes de silicone, conformes à la présente invention, sont des polydiorganosiloxanes de forte masse moléculaire comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On cite, par exemple, les composés suivants :
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)].

On peut citer, par exemple, à titre non limitatif, les mélanges suivants :
1/ les mélanges formés à partir d'un polydiméthylsiloxane hydroxylé en bout de chaîne (DIMETHICO-NOL selon la nomenclature CTFA), et d'un polydiméthylsiloxane cyclique (CYCLOMETHICONE selon la nomenclature CTFA), tels que le produit Q2 1401 vendu par la Société DOW CORNING;
2/ les mélanges formés à partir d'une gomme polydiméthylsiloxane avec une silicone cyclique, tel que le produit SF 1214 SILICONE FLUID de GENERAL ELECTRIC, qui est une gomme SE 30 de PM 500.000 solubilisée dans la SF 1202 SILICONE FLUID (décaméthylcyclopentasiloxane);
3/ les mélanges de deux PDMS de viscosités différentes, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus d'une viscosité de 20 m²/s et d'une huile SF 96 d'une viscosité de 5.10⁻⁶ m²/s (15% de gomme SE 30 et 85% d'huile SF 96).

Le produit CF 1241 est le mélange d'une gomme SE 30 (33%) et d'une PDMS (67%) de viscosité 10⁻³ m²/s_{.}

Les résines d'organopolysiloxanes utilisables conformément à l'invention, sont des systèmes siloxaniques réticulés renfermant les unités :
R₂SiO_{2/2}, RSiO_{3/2} et SiO_{4/2} dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination DOW CORNING 593 ou ceux vendus sous les dénominations SILICONE FLUID SS 4230 et SS 4267 par la Société GENERAL ELECTRIC et qui sont des "diméthyl/triméthylpolysiloxane".

Les silicones organomodifiées conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant :
a) des groupements polyéthylèneoxy et/ou polypropylèneoxy, comportant éventuellement des groupes alkyle, tels que :
   - le produit dénommé diméthicone copolyol vendu par la Société DOW CORNING sous les dénominations DC 1248, et l'alkyl(C12)méthicone copolyol vendue par la Société DOW CORNING sous la dénomination Q2 5200,
   - les huiles SILWET L 722, L 7500, L 77, L 711 de la Société UNION CARBIDE,
   - le mélange de diméthicone copolyol et de cyclométhicone tels que le produit vendu sous la dénomination Q2-3225C par la Société DOW CORNING;
b) des groupements perfluorés tels que des trifluoroalkyles telles que, par exemple, celles vendues par la Société GENERAL ELECTRIC sous les dénominations "FF.150 Fluorosilicone Fluid" ou par la Société SHIN ETSU sous les dénominations X-22-819; X-22-820; X-22-821; X-22-822;
c) des groupements hydroxyacylamino telles que celles décrites dans la demande de brevet européen EPA 0342834 et en particulier la silicone vendue par la Société DOW CORNING sous la dénomination Q2-8413;
d) des groupements thiols comme dans les silicones X 2-8360 de la DOW CORNING ou les GP 72A et GP 71 de GENESEE;
e) des groupements aminés substitués ou non, comme dans la GP4 SILICONE FLUID de GENESEE, la GP 7100 de GENESEE, la Q2 8220 de DOW CORNING, l'AFL 40 d'UNION CARBIDE ou la silicone dénommée "Amodiméthicone" dans le dictionnaire CTFA;
f) des groupements carboxylates, comme les produits décrits dans le brevet européen EP 186 507 de CHISSO CORPORATION;
g) des groupements hydroxylés, comme les polyorganosiloxanes à fonction hydroxyalkyle, décrits dans la demande de brevet en France n • FR-85 16334, répondant à la formule suivante : 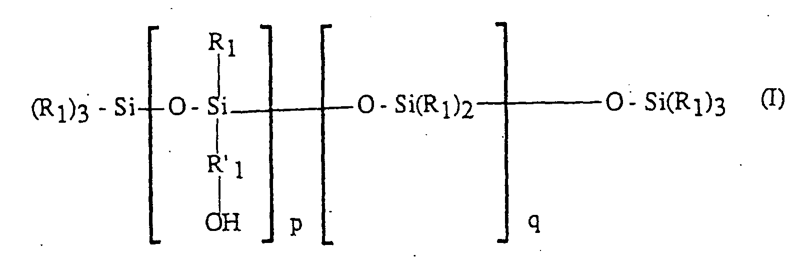 dans laquelle
   - les radicaux R₁, identiques ou différents, sont choisis parmi les radicaux méthyle et phényle au moins 60% en mole des radicaux R₁ étant méthyle;
   - le radical R'₁ est un chaînon alkylène divalent hydrocarboné en C₂-C₁₈;
   - p est compris entre 1 et 30 inclus;
   - q est compris entre 1 et 150 inclus;
h) des groupements alcoxylés comme dans la Silicone copolymer F 755 de SWS SILICONES et les produits ABILWAX 2428, ABILWAX 2434, ABILWAX 2440 de la Société GOLDSCHMIDT;
i) des groupements acyloxyalkyle, comme par exemple les polyorganopolysiloxanes décrits dans la demande de brevet français n •88 17433, répondant à la formule suivante : 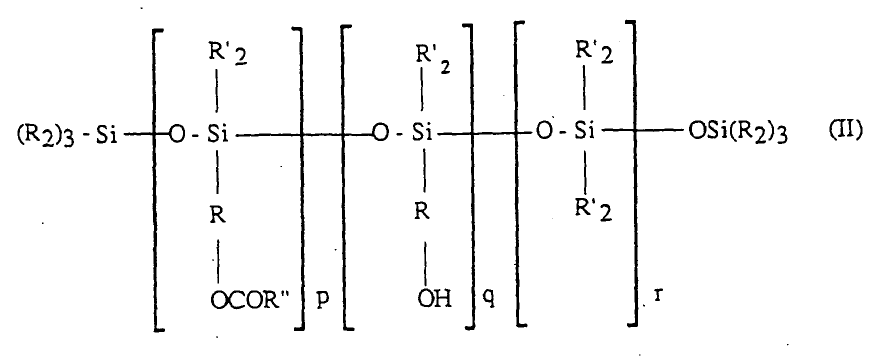 dans laquelle :
   - R₂ désigne méthyle, phényle, OCOR", hydroxyle, un seul des R₂ par atome de silicium peut être OH;
   - R'₂ désigne méthyle, phényle, 60% molaire au moins de l'ensemble des radicaux R₂ et R'₂ est méthyle;
   - R" désigne alcoyle ou alcényle en C₈-C₂₀;
   - R désigne un alkylène hydrocarboné divalent, linéaire ou ramifié, en C₂-C_{18;}
   - r est compris entre 1 et 120 inclus;
   - p est compris entre 1 et 30;
   - q vaut 0 ou est inférieur à 0,5 p, p + q étant compris entre 1 et 30; les polyorganosiloxanes de formule (II) peuvent contenir des groupements 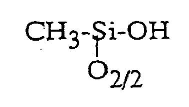 dans des proportions ne dépassant pas 15% de la somme p + q + r;
j) des groupements ammonium quaternaire, comme dans les produits X2 81 08 et X2 81 09, le produit ABIL K 3270 de la Société GOLDSCHMIDT;
k) des groupements amphotères ou bétaïniques, tels que dans le produit vendu par la Société GOLDSCHMIDT sous la dénomination ABIL B 9950;
l) des groupements bisulfite, tels que dans les produits vendus par la Société GOLDSCHMIDT sous les dénominations ABIL S 201 et ABIL S 255.

Les polyorganosiloxanes particulièrement préférés, selon la présente invention, sont choisis parmi :
1) les silicones non volatiles du type polyalkylsiloxane linéaire à groupements terminaux triméthylsilyle, telles que les huiles SILBIONE des séries 70047 et 47 telles que l'huile 47 V 500.000 commercialisées par RHONE POULENC ou du type polyalkylarylsiloxane comme l'huile Silbione 70641 V 200 de RHONE POULENC;
2) les mélanges d'organosiloxanes et de silicones cycliques tels que la Q2 1401 de la Société DOW CORNING, la SF 1214 SILICONE FLUID de la Société GENERAL ELECTRIC;
3) les fluorosilicones de type polyalkylsiloxane à groupements terminaux triméthylsilyle et substituées sur la chaîne par des groupements trifluoropropyle telle que la fluorosilicone vendue par la Société SHIN ET SU sous la dénomination X-22-821.

Les polyorganosiloxanes utilisés conformément à la présente invention, sont présents dans la dispersion aqueuse dans une proportion comprise entre 0,5 et 50% en poids et de préférence entre 1 et 30% en poids, par rapport au poids total de la dispersion.

Le copolymère réticulé d'acrylamide/acide 2-acrylamido 2-méthyl propane sulfonique utilisé conformément à la présente invention, est plus particulièrement un copolymère réticulé par un composé à polyinsaturation oléfinique tel que le tétraallyloxyéthane, l'allylsucrose, l'allylpentaérythritol ou le méthylène bis-acrylamide, partiellement ou totalement neutralisé par un agent de neutralisation tel que la soude, la potasse, l'ammoniaque ou une amine telle que la triéthanolamine ou la monoéthanolamine.

Les copolymères de l'invention peuvent être préparés en copolymérisant l'acrylamide et le 2-acrylamido 2-méthylpropane sulfonate de sodium par voie radicalaire au moyen d'agents initiateurs du type azobiisobutyronitrile et par précipitation dans un alcool tel que le tertiobutanol.

Les copolymères préférentiels sont obtenus par copolymérisation de 70 à 55% en moles d'acrylamide et de 30 à 45% en moles de 2-acrylamido 2-méthylpropane sulfonate de sodium; l'agent de réticulation étant utilisé à des concentrations de 10⁻⁴ à 4.10⁻⁴ mole par mole du mélange des monomères.

Les copolymères sont présents dans les dispersions aqueuses de l'invention à des concentrations comprises entre 0,05 et 10% en poids et de préférence entre 0,1 et 6% en poids.

Une forme particulièrement préférée de dispersion aqueuse utilisée pour le traitement cosmétique des cheveux conformément à l'invention, consiste à mettre en oeuvre une dispersion aqueuse contenant :
a) un organopolysiloxane;
b) le copolymère réticulé d'acrylamide/acide 2-acrylamido 2-méthylpropane sulfonique neutralisé;
c) un agent émulsionnant non-ionique;
d) un ou plusieurs hydrocarbures isoparaffiniques de point d'ébullition élevé.

Le ou les hydrocarbures isoparaffiniques sont présents dans des proportions de préférence comprises entre 0,02 et 6,5% en poids par rapport au poids total des dispersions.

On utilise en particulier un mélange d'hydrocarbures isoparaffiniques en C₁₂-C₁₃ tel que le produit vendu sous le nom ISOPAR M par la Société EXXON CHEMICALS.

Des agents émulsionnants non-ioniques utilisés selon l'invention sont choisis par exemple parmi les esters d'acides gras de sorbitan, les esters d'acides gras, les esters d'acides gras éthoxylés, les alcools gras et les alcools gras éthoxylés, les copolymères séquencés du type oxyde d'éthylène/oxyde de propylène ou oxyde d'éthylène/oxyde de butylène, ou leurs mélanges.

Les agents émulsionnants sont de préférence présents dans des proportions comprises entre 0,01 et 1,5% en poids par rapport au poids total des dispersions. On utilise de préférence le lauryléther de polyéthylèneglycol à 7 moles d'oxyde d'éthylène.

Une forme particulièrement préférée de dispersion aqueuse utilisée selon l'invention contient une émulsion huile-dans-eau constituée par 35 à 45% en poids de copolymère réticulé d'acrylamide/acide 2-acrylamido 2-méthylpropane sulfonique neutralisé; 15 à 25% d'hydrocarbures isoparaffiniques; 3 à 8% en poids de lauryléther de polyéthylèneglycol à 7 moles d'oxyde d'éthylène et d'eau. Une telle émulsion est commercialisée sous le nom de SEPIGEL 305 par la Société SEPPIC.

Cette dispersion aqueuse particulière, utilisée selon l'invention, est de préférence préparée par simple mélange à température ambiante et sous agitation du polymère organosiloxane avec l'émulsion telle que définie ci-dessus. Le mélange ainsi obtenu peut être directement introduit dans l'eau renfermant d'autres ingrédients choisis en fonction de l'application désirée.

L'émulsion huile-dans-eau renfermant le copolymère particulier de l'invention tel que défini ci-dessus, est présente dans la dispersion aqueuse dans des proportions telles que la concentration en copolymère soit comprise entre 0,05 et 10% en poids et de préférence 0,1 et 6% en poids de matière active de copolymère, par rapport au poids total de la dispersion.

Un autre objet de l'invention est constitué par une composition sous forme de dispersion aqueuse destinée au traitement des cheveux en cosmétique, caractérisée par le fait que la dispersion aqueuse est telle que définie précédemment.

Les compositions conformes à la présente invention peuvent contenir en plus des adjuvants habituellement utilisés en cosmétique tels que des parfums, des colorants, des conservateurs, des agents séquestrants, des huiles végétales, animales ou synthétiques, des filtres solaires, des agents tensio-actifs anioniques, non-ioniques, amphotères ou cationiques, des polymères, des protéines, des agents de conditionnement, des stabilisateurs de mousse, des propulseurs ou autres adjuvants habituellement utilisés dans les compositions pour les cheveux, suivant l'application envisagée.

Les compositions cosmétiques destinées au traitement des cheveux, conformes à l'invention, peuvent être utilisées en particulier comme shampooing, comme produit à rincer, à appliquer avant ou après shampooing, avant, pendant ou après coloration ou décoloration, avant ou après permanente ou défrisage ou en lotion intrapermanente ou comme produit coiffant non rincé, tel que dans des lotions de mises en plis ou de brushing.

De façon préférentielle, lorsque les compositions selon l'invention contiennent des tensio-actifs détergents, ceux-ci sont présents en une proportion inférieure à 5% en poids par rapport au poids total de la composition.

Les compositions cosmétiques selon la présente invention présentent un pH compris entre 3 et 10 et de préférence entre 5 et 7. Ce pH peut être ajusté par des agents alcalinisant ou acidifiants habituellement utilisés en cosmétique.

Un procédé de traitement cosmétique des cheveux selon l'invention consiste à appliquer les compositions telles que définies ci-dessus sur les cheveux suivant l'usage envisagé (shampooing, traitement à rincer, traitement de coiffage sans rinçage), sans qu'il soit nécessaire d'observer un temps de pose et à rincer éventuellement.

Les exemples qui suivent sont destinés à illustrer la présente invention, sans pour autant présenter un caractère limitatif.

### EXEMPLE 1

On prépare une composition de soin non rincée :
- Emulsion huile-dans-eau de copolymère réticulé d'acrylamide/2-acrylamido 2-méthyl propane sulfonate de sodium, vendue par la Société SEPPIC sous la dénomination SEPIGEL 305 0,17 g en copolymère
- Mélange de diméthiconol (13%) d'octaméthylcyclotétrasiloxane et de décaméthylcyclopentasiloxane (87%), vendu sous la dénomination Q2-1401 par la Société DOW CORNING 15 g
- Polydiorganosiloxane à fonction thiol de formule : 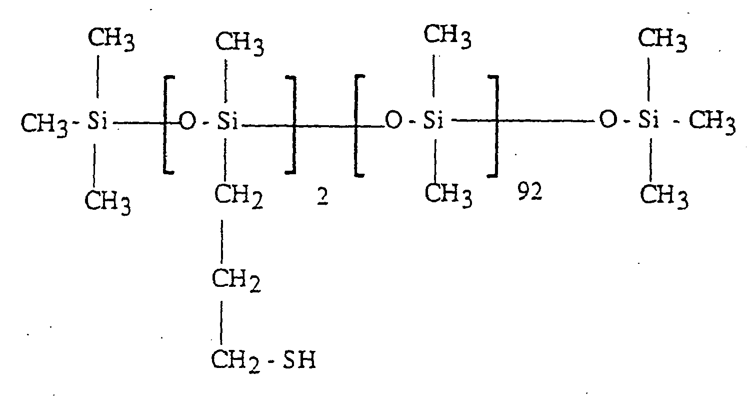 vendu sous la dénomination X2 8360 par la Société DOW CORNING 5 g
- Conservateur, parfum qs
- pH spontané = 6.3
- Eau qsp 100 g

### EXEMPLE 2

On prépare une composition de soin non rincé :

| | |
|---|---|
| - Emulsion huile-dans-eau de copolymère réticulé d'acrylamide/2-acrylamido 2-méthyl propane sulfonate de sodium, vendue par la Société SEPPIC sous la dénomination SEPIGEL 305 | 0,14 g en copolymère |
| - Polydiméthylméthyltrifluoropropylsiloxane, vendu par la Société SHIN ETSU sous la dénomination X-22-821 | 20 g |
| - Conservateur, parfum qs | |
| - pH spontané = 7,2 | |
| - Eau qsp | 100 g |

### EXEMPLE 3

On prépare une composition de soin non rincé :

| | |
|---|---|
| - Emulsion huile-dans-eau de copolymère réticulé d'acrylamide/2-acrylamido 2-méthyl propane sulfonate de sodium, vendue par la Société SEPPIC sous la dénomination SEPIGEL 305 | 0,35 g en copolymère |
| - Polydiméthylsiloxane (PM 250.000), vendu sous la dénomination 47 V 500.000 par la Société DOW CORNING | 5 g |
| - Conservateur, parfum qs | |
| - HCl qs pH=5 | |
| - Eau qsp | 100 g |

### EXEMPLE 4

On prépare un après-shampooing à rincer de composition suivante :
- Emulsion huile-dans-eau de copolymère réticulé d'acrylamide/2-acrylamido 2-méthyl propane sulfonate de sodium, vendue par la Société SEPPIC sous la dénomination SEPIGEL 305 0,21 g en copolymère
- Polydiorganosiloxane de formule : 5 g 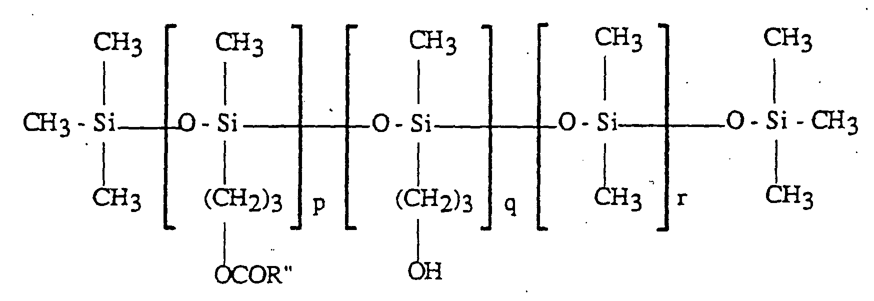 R" = mélange de radicaux C₁₆H₃₃ et C₁₈H₃₇
   p = 7,9 ; q=1,4; r=9,3

Ce composé comporte en moyenne 2 motifs

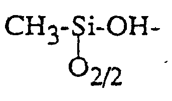

Il peut être préparé comme décrit à l'exemple A de la demande FR-2.641.185.
- Conservateur, parfum qs
- pH spontané = 7,5
- Eau qsp 100 g

### EXEMPLE 5

On prépare un après-shampooing à rincer de composition suivante :

| | |
|---|---|
| - Emulsion huile-dans-eau de copolymère réticulé d'acrylamide/2-acrylamido 2-méthyl propane sulfonate de sodium, vendue par la Société SEPPIC sous la dénomination SEPIGEL 305 | 0,14 g en copolymère |
| - Mélange de diméthiconol (13%), d'octaméthylcyclotétrasiloxane et de décaméthylcyclopentasiloxane (87%), vendu sous la dénomination Q2-1401 par la Société DOW CORNING | 20 g |
| - Conservateur, parfum qs | |
| - pH spontané = 7,6 | |
| - Eau qsp | 100 g |

### EXEMPLE 6

On prépare un après-shampooing de composition suivante :

| | |
|---|---|
| - Emulsion huile-dans-eau de copolymère acrylamide/acrylamido 2-méthyl propane sulfonate de sodium, vendue par la Société SEPPIC sous la dénomination SEPIGEL 305 | 1,5 g en copolymère |
| - Micro-émulsion non-ionique à 30% d'huile de silicone aminée (amodiméthicone de viscosité 3.10⁻⁴ m²/s), vendue par la Société RHONE POULENC | 3 g en silicone |
| - Conservateur, parfum qs | |
| - pH spontané = 6,5 | |
| - Eau qsp | 100 g |

### EXEMPLE 7

On prépare un après-shampooing de composition suivante :

| | |
|---|---|
| - Emulsion huile-dans-eau de copolymère acrylamide/acrylamido 2-méthyl propane sulfonate de sodium, vendue par la Société SEPPIC sous la dénomination SEPIGEL 305 | 2 g en copolymère |
| - Mélange de diméthiconol (13%), d'octaméthylcyclotétrasiloxane et de décaméthylcyclopentasiloxane (87%), vendu par la Société DOW CORNING sous la dénomination 02-1401 | 5 g |
| - Polymère d'hydroxyéthylcellulose et d'épichlorhydrine quaternisé avec la triméthylamine, vendu par la Société UNION CARBIDE sous la dénomination JR 400 | 1 g |
| - Conservateur, parfum qs | |
| - Triéthanolamine qs pH = 6.5 | |
| - Eau qsp | 100 g |

### EXEMPLE 8

On prépare une composition d'après-shampooing à rincer :

| | |
|---|---|
| - Emulsion huile-dans-eau de copolymère acrylamide/acrylamido 2-méthyl propane sulfonate de sodium, vendue par la Société SEPPIC sous la dénomination SEPIGEL 305 | 10 g en copolymère |
| - Mélange de deux polydiméthylsiloxanes de viscosités différentes, vendu par la Société GENERAL ELECTRIC sous la dénomination CF 1241 | 4,5 g |
| - Conservateur qs | |
| - pH spontané = 5,5 | |
| - Eau qsp | 100 g |

### EXEMPLE 9

On prépare une composition d'après-shampooing à rincer :

| | |
|---|---|
| - Emulsion huile-dans-eau de copolymère acrylamide/acrylamido 2-méthyl propane sulfonate de sodium, vendue par la Société SEPPIC sous la dénomination SEPIGEL 305 | 5 g en copolymère |
| - Polydiméthylsiloxane (PM 250.000) vendu par la Société DOW CORNING sous la dénomination 70047 V 500.000 | 3 g |
| - Conservateur qs | |
| - Triéthanolamine qs pH = 7,2 | |
| - Eau qsp | 100 g |

## Revendications

1. Utilisation pour le traitement cosmétique des cheveux, d'une dispersion aqueuse, **caractérisée par le fait que** ladite dispersion contient au moins, dans un milieu aqueux cosmétiquement ou physiologiquement acceptable, un organopolysiloxane et un copolymère réticulé d'acrylamide/acide 2-acrylamido 2-méthylpropane sulfonique neutralisé: sous réserve que l'organopolysiloxane ne soit pas choisi parmi les polydiméthylsiloxanes linéaires de viscosité inférieure à 10⁻¹ m²/s.

2. Utilisation selon la revendication 1. **caractérisée par le fait que** l'organosiloxane est une silicone volatile ayant un point d'ébullition compris entre 60°C et 260°C, choisie parmi :
(i) les silicones cycliques de 3 à 7 atomes de silicium et de préférence 4 à 5 ou les cyclocopolymères du type diméthylsiloxane/méthylalkylsiloxane.
(ii) les silicones linéaires, ayant 2 à 9 atomes de silicium et possédant une viscosité inférieure ou égale à 5.10⁻⁶ m²/s à 25°C.

3. Utilisation selon l'une quelconque des revendications 1 à 2, **caractérisée par le fait que** l'organopolysiloxane est une silicone non volatile choisie parmi les polyalkylsiloxanes, les polyarylsiloxanes, les polyalkylarylsiloxanes, les gommes et résines de silicone, les polysiloxanes organomodifiés, ainsi que leurs mélanges.

4. Utilisation selon la revendication 3, **caractérisée par le fait que** l'organopolysiloxane est choisi parmi :
A/ les polyalkyl(C₁-C₂₀)siloxanes: les polydiméthylsiloxanes linéaires à groupements terminaux triméthylsilyle et les polydiméthylsiloxanes linéaires à groupements terminaux trihydroxysilyle, de viscosité supérieure à 10⁻¹ m²/s:
B/ les polydiméthylphénylsiloxanes, les polydiméthyldiphénylsiloxanes linéaires et ou ramifiés, de viscosité 10⁻⁵ à 5.10⁻² m²/s à 25°C:
C/ les gommes de masse moléculaire comprise entre 200:000 et 1.000.000. utilisées seules ou sous forme de mélange dans un solvant, choisies dans le groupe constitué par les copolymères suivants :
- poly[(diméthylsiloxane) (méthylvinylsiloxane)].
- poly[(diméthylsiloxane) (diphénylsiloxane)],
- poly[(diméthylsiloxane)(phénylméthylsiloxane)].
- poly[(diméthylsiloxane)(diphénylsiloxane)(méthylvinylsiloxane)]:et les mélanges suivants :
- les mélanges formés à partir d'un polydiméthylsiloxane hydroxylé en bout de chaîne et d'un polydiméthylsiloxane cyclique:
- les mélanges formés à partir d'une gomme polydiméthylsiloxane et d'une silicone cyclique:
- les mélanges de deux polydiméthylsiloxanes de viscosités différentes:
D/ les résines d'organopolysiloxanes renfermant les unités R₂SiO₂₂, RSiO_{3/2} et SiO_{4/2} dans lesquel les R représente un groupement hydrocarboné possédant 1 à 6 atomes de carbone ou un groupement phényle.

5. Utilisation selon la revendication 3, **caractérisée par le fait que** l'organopolysiloxane comporte dans sa structure générale un ou plusieurs groupement(s) organofonctionnel(s) directement fixé(s) sur la chaîne siloxanique ou fixé(s) par l'intermédiaire d'un radical hydrocarboné, et qu'il est choisi parmi les polyorganosiloxanes comportant :
a) des groupements polyéthylèneoxy et ou polypropylèneoxy.
b) des groupes perfluorés:
c) des groupes hydroxyacylamino:
d) des groupements thiol:
e) des groupes aminés substitués ou non:
f) des groupements carboxylates:
g) des groupements hydroxyalkyle de formule : 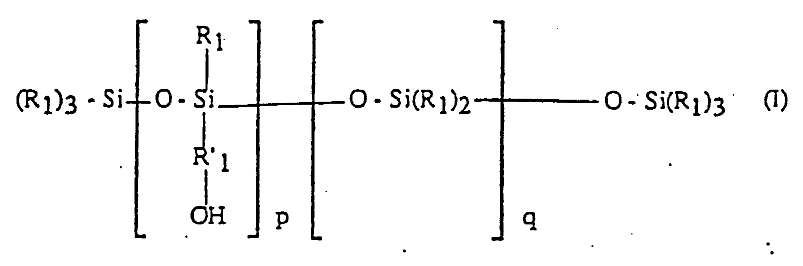 dans laquelle :
. les radicaux R₁, identiques ou différents, sont choisis parmi les radicaux méthyle et phényle au moins 60% en mole des radicaux R₁ étant méthyle:
. le radical R'₁ est un chaînon alkylène divalent hydrocarboné en C₂-C₁₈;
. p est compris entre 1 et 30 inclus;
. q est compris entre 1 et 150 inclus:
h) des groupements alcoxylés:
i) des groupements acyloxyalkyle répondant à la formule suivante : 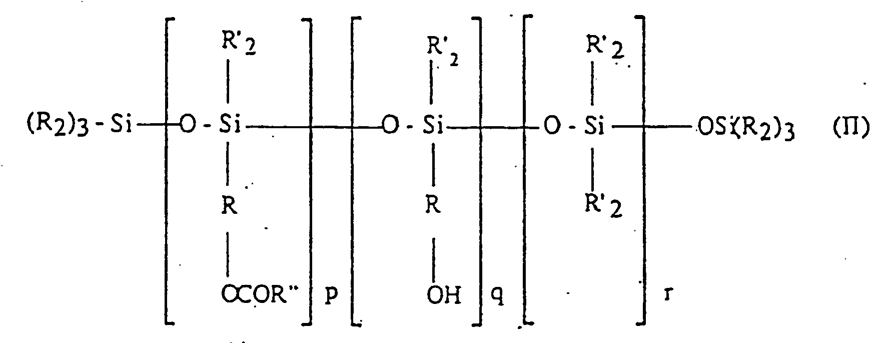 dans laquelle :
. R₂ désigne méthyle, phényle, OCOR", hydroxyle, un seul des R₂ par atome de silicium peut être OH;
. R'₂ désigne méthyle, phényle, 60% molaire au moins de l'ensemble des radicaux R₂ et R'₂ est méthyle:
. R" désigne alcoyle ou alcényle en C₉-C₂₀:
. R désigne un alkylène hydrocarboné divalent, linéaire ou ramifié, en C₂-C₁₂;
. r est compris entre 1 et 120 inclus:
. p est compris entre 1 et 30:
. q vaut 0 ou est inférieur à 0.5 p, p + q étant compris entre 1 et 30; les polyorganosiloxanes de formule (II) peuvent contenir des groupements 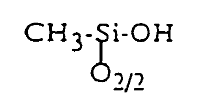 dans des proportions ne dépassant pas 15% de la somme p + q + r;
j) des groupements ammonium quaternaire;
k) des groupements amphotères ou bétaïniques:
l) des groupements bisulfite.

6. Utilisation selon l'une quelconque des revendications 1 à 5, **caractérisée par le fait que** l'organopolysiloxane est choisi parmi :
1) les silicones non volatiles du type polyalkylsiloxane linéaire à groupements terminaux triméthylsilyle ou du type polyalkylphénylsiloxane;
2) les mélanges d'organosiloxanes et de silicones cycliques;
3) les fluorosilicones de type polyalkylsiloxane à groupements terminaux triméthylsilyle et substituées sur la chaîne par des groupements trifluoropropyle.

7. Utilisation selon l'une quelconque des revendications 1 à 6, **caractérisée par le fait que** le copolymère d'acrylamide acide 2-acrylamido 2-méthylpropane sulfonique est réticulé par un agent de réticulation à polyinsaturation oléfinique choisi parmi le tétraallyloxyéthane, l'allylsucrose, l'allylpentaérythritol, le méthylène bis-acrylamide et qu'il est partiellement ou totalement neutralisé par de la soude, de la potasse, de l'ammoniaque ou une amine.

8. Utilisation selon la revendication 7, **caractérisée par le fait que** le copolymère est obtenu par copolymérisation de 70 à 55% en moles d'acrylamide et 30 à 45% en moles de 2-acrylamido 2-méthylpropane sulfonate de sodium en présence de 10⁻⁴ à 4.10⁻⁴ mole d'agent de réticulation par mole de mélange des deux monomères.

9. Utilisation selon l'une quelconque des revendications 1 à 8, **caractérisée par le fait que** l'organopolysiloxane est présent dans des proportions comprises entre 0,5 et 50% en poids par rapport au poids total de la dispersion et le copolymère est présent dans des proportions comprises entre 0,05 et 10% en poids.

10. Utilisation selon l'une quelconque des revendications 1 à 9, **caractérisée par le fait que** la dispersion aqueuse comprend :
a) l'organopolysiloxane;
b) le copolymère réticulé acrylamide acide 2-acrylamido 2-méthyl propane sulfonique neutralisé;
c) un agent émulsionnant non-ionique:
d) un hydrocarbure ou un mélange d'hydrocarbures isoparaffinique(s) de point d'ébullition élevé.

11. Utilisation selon la revendication 10, **caractérisée par le fait que** l'agent émulsionnant est choisi parmi les esters d'acides gras du sorbitan, les esters d'acides gras éthoxylés ou non, les alcools gras éthoxylés ou non, les copolymères séquencés du type oxyde d'éthylène oxyde de propylène ou oxyde d'éthylène oxyde de butylène, ou leurs mélanges.

12. Utilisation selon la revendication 10 ou 11, **caractérisée par le fait que** l'agent émulsionnant est présent dans des proportions comprises entre 0.01 et 1.5% en poids, que le ou les hydrocarbures isoparaffiniques sont présents dans des proportions comprises entre 0,02 et 6.5% en poids par rapport au poids total de la dispersion.

13. Utilisation selon l'une quelconque des revendications 10 à 12. **caractérisée par le fait que** l'agent émulsionnant non-ionique est le lauryléther de polyéthylèneglycol à 7 moles d'oxyde d'éthylène et qu'on utilise un mélange d'hydrocarbures isoparaffiniques en C₁₂-C₁₃.

14. Utilisation selon l'une quelconque des revendications 1 à 13, **caractérisée par le fait que** le copolymère réticulé d'acrylamide acide 2-acrylamido 2-méthylpropane sulfonique neutralisé est dispensé à raison de 35 à 45% en poids dans une émulsion huile-dans-eau contenant 15 à à 25% en poids de mélange d'hydrocarbures isoparaffiniques en C₁₂-C₁₃. de 3 à 8% en poids de lauryléther de polyéthylèneglycol à 7 moles d'oxyde d'éthylène et de l'eau.

15. Utilisation selon la revendication 14, **caractérisée par le fait que** l'émulsion huile-dans-eau contenant le copolymère réticulé acrylamide acide 2-acrylamido 2-méthylpropane sulfonique neutralisé est présente dans la dispersion aqueuse dans des proportions telles que la concentration en copolymère soit comprise entre 0,05 et 10% en poids de matière active par rapport au poids total de la dispersion.

16. Composition cosmétique sous forme de dispersion aqueuse destinée au traitement des cheveux,
**caractérisée par le fait que** la dispersion aqueuse est telle que définie dans l'une quelconque des revendications 1 à 15.

17. Composition selon la revendication 16, **caractérisée par le fait qu'**elle contient en plus des adjuvants habituellement utilisés en cosmétique, choisis parmi les parfums, les colorants, les conservateurs, des huiles végétales, animales ou synthétiques, des protéines, des agents de conditionnement, les agents tensio-actifs anioniques, non-ioniques, amphotères ou cationiques, les séquestrants, les stabilisateurs de mousse, des polymères, des filtres solaires, des propulseurs, des substances actives sur le plan cosmétique.

18. Composition selon l'une des revendications 16 ou 17, **caractérisée par le fait qu'**elle présente un pH compris entre 3 et 10 et de préférence entre 5 et 7.

19. Composition selon l'une quelconque des revendications 16 à 18, destinée au traitement des cheveux, **caractérisée par le fait qu'**elle se présente sous forme de shampooing, de produit à rincer, à appliquer avant ou après shampooing, avant, pendant ou après coloration ou décoloration, avant ou après permanente ou défrisage, en lotion intrapermanente, ou comme produits de coiffage non rincés.

20. Procédé de traitement cosmétique des cheveux, **caractérisé par le fait que** l'on applique sur ceux-ci au moins une composition telle que définie dans la revendication 19.

## Claims

1. Use in cosmetic hair treatment of an aqueous dispersion, **characterized in that** the said dispersion contains, in a cosmetically or physiologically acceptable aqueous medium, at least one organopolysiloxane and one crosslinked acrylamide/neutralized 2-acrylamido-2-methylpropanesulfonic acid copolymer, with the proviso that the organopolysiloxane is not chosen from linear polydimethylsiloxanes with a viscosity of less than 10⁻¹ m²/s.

2. Use according to Claim 1, **characterized in that** the organosiloxane is a volatile silicone having a boiling point between 60°C and 260°C, chosen from:
(i) cyclic silicones containing 3 to 7 silicon atoms and preferably 4 to 5 or cyclocopolymers of dimethylsiloxane/methylalkylsiloxane type,
(ii) linear silicones having 2 to 9 silicon atoms and having a viscosity less than or equal to 5.10⁻⁶ m²/s at 25°C.

3. Use according to any one of Claims 1 to 2, **characterized in that** the organopolysiloxane is a nonvolatile silicone chosen from polyalkylsiloxanes, polyarylsiloxanes, polyalkylarylsiloxanes, silicone gums and resins and organomodified polysiloxanes, as well as their mixtures.

4. Use according to Claim 3, **characterized in that** the organopolysiloxane is chosen from:
A) poly(C₁-C₂₀)alkylsiloxanes, linear polydimethylsiloxanes containing trimethylsilyl end groups and linear polydimethylsiloxanes containing trihydroxysilyl end groups, with a viscosity greater than 10⁻¹ m²/S;
B) linear and/or branched polydimethyldiphenylsiloxanes or polydimethylphenylsiloxanes, with a viscosity 10⁻⁵ to 5.10⁻² m²/s at 25°C.
C) gums with a molecular mass of between 200,000 and 1,000,000, used alone or in the form of a mixture in a solvent, chosen from the group consisting of the following copolymers:
- poly[dimethylsiloxane/methylvinylsiloxane],
- poly[dimethylsiloxane/diphenylsiloxane],
- poly[dimethylsiloxane/phenylmethylsiloxane],
- poly[dimethylsiloxane/diphenylsiloxane/methylvinylsiloxane], and the following mixtures:
- mixtures formed from a polydimethylsiloxane hydroxylated at the chain ends and from a cyclic polydimethylsiloxane,
- mixtures formed from a polydimethylsiloxane gum and from a cyclic silicone,
- mixtures of two polydimethylsiloxanes having different viscosities;
D) organopolysiloxane resins containing R₂SiO_{2/2}, RSiO_{3/2} and SiO_{4/2} units in which R represents a hydrocarbon group having 1 to 6 carbon atoms or a phenyl group.

5. Use according to Claim 3, **characterized in that** the organopolysiloxane contains in its general structure one or a number of organofunctional group(s) attached directly to the siloxane chain or attached via a hydrocarbon radical, and **in that** it is chosen from polyorganosiloxanes containing:
a) polyoxyethylene and/or polyoxypropylene groups,
b) perfluorinated groups,
c) hydroxyacylamino groups,
d) thiol groups,
e) substituted or unsubstituted amino groups,
f) carboxylate groups,
g) hydroxyalkyl groups of formula: 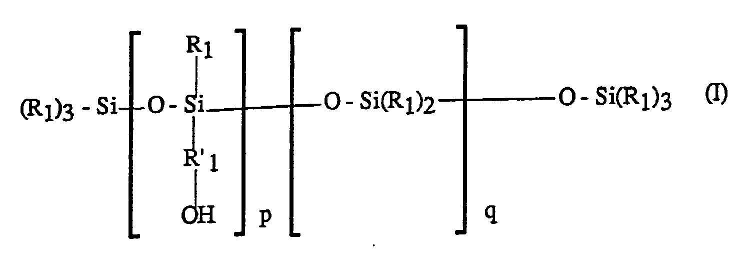 in which
- the radicals R₁, which are identical or different, are chosen from methyl and phenyl radicals, at least 60 mol% of the radicals R₁ being methyl,
- the radical R'₁ is a divalent C₂-C₁₈ alkylene hydrocarbon chain,
- p is between 1 and 30 inclusive;
- q is between 1 and 150 inclusive;
h) alkoxylated groups,
i) acyloxyalkyl groups corresponding to the following formula: 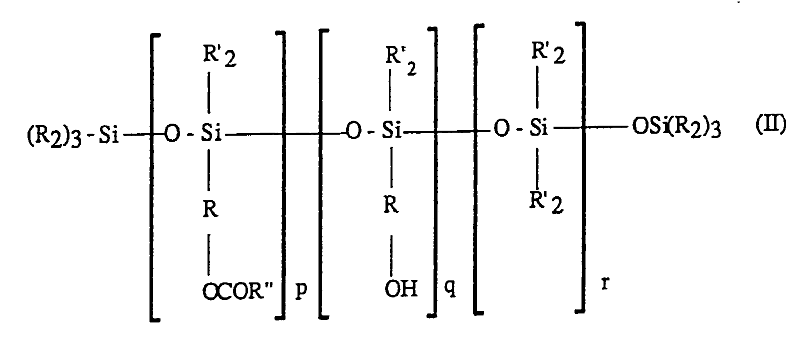 in which:
- R₂ denotes methyl, phenyl, OCOR " or hydroxyl, a single one of the R₂ groups per silicon atom can be OH,
- R'₂ denotes methyl or phenyl, at least 60 mol % of the combined radicals R₂ and R'₂ is [sic] methyl,
- R'' denotes C₈-C₂₀ alkyl or alkenyl,
- R denotes a linear or branched, divalent C₂-C₁₈ alkylene hydrocarbon,
- r is between 1 and 120 inclusive,
- p is between 1 and 30,
- q has the value 0 or is less than 0.5 p, p + q being between 1 and 30: the polyorganosiloxanes of formula (II) can contain 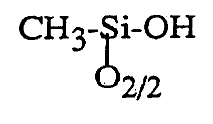 groups in proportions not exceeding 15% of the sum p + q + r,
j) quaternary ammonium groups,
k) amphoteric or betaine groups,
l) bisulfite groups.

6. Use according to any one of Claims 1 to 5, **characterized in that** the organopolysiloxane is chosen from:
1) nonvolatile silicones of linear polyalkylsiloxane type containing trimethylsilyl end groups or of polyalkylphenylsiloxane type,
2) mixtures of organosiloxanes and of cyclic silicones,
3) fluorosilicones of polyalkylsiloxane type containing trimethylsilyl end groups and substituted on the chain by trifluoropropyl groups.

7. Use according to any one of Claims 1 to 6, **characterized in that** the acrylamide/2-acrylamido-2-methylpropanesulfonic acid copolymer is crosslinked with a crosslinking agent containing olefinic polyunsaturation chosen from tetraallyloxyethane, allylsucrose, allylpentaerythritol or methylenebisacrylamide and **in that** it is partially or completely neutralized by sodium hydroxide, potassium hydroxide, aqueous ammonia or an amine.

8. Use according to Claim 7, **characterized in that** the copolymer is obtained by copolymerization of 70 to 55 mol % of acrylamide and 30 to 45 mol % of sodium 2-acrylamido-2-methylpropanesulfonate in the presence of 10⁻⁴ to 4.10⁻⁴ mol of crosslinking agent per mole of mixture of the two monomers.

9. Use according to any one of Claims 1 to 8, **characterized in that** the organopolysiloxane is present in proportions of between 0.5 and 50% by weight with respect to the total weight of the dispersion and the copolymer is present in proportions of between 0.05 and 10% by weight.

10. Use according to any one of Claims 1 to 9, **characterized in that** the aqueous dispersion comprises:
a) the organopolysiloxane,
b) the crosslinked acrylamide/neutralized 2-acrylamido-2-methylpropanesulfonic acid copolymer,
c) a nonionic emulsifying agent,
d) an isoparaffin hydrocarbon or a mixture of isoparaffin hydrocarbons having a high boiling point.

11. Use according to Claim 10, **characterized in that** the emulsifying agent is chosen from sorbitan fatty acid esters, ethoxylated or non-ethoxylated fatty acid esters, ethoxylated or non-ethoxylated fatty alcohols, or block copolymers of ethylene oxide/propylene oxide or ethylene oxide/butylene oxide type, or their mixtures.

12. Use according to Claim 10 or 11, **characterized in that** the emulsifying agent is present in proportions of between 0.01 and 1.5% by weight and **in that** the isoparaffin hydrocarbon(s) are present in proportions of between 0.02 and 6.5% by weight with respect to the total weight of the dispersion.

13. Use according to any one of Claims 10 to 12, **characterized in that** the nonionic emulsifying agent is the ether of lauryl alcohol and of polyethylene glycol containing 7 mol of ethylene oxide and **in that** a mixture of C₁₂-C₁₃ isoparaffin hydrocarbons is used.

14. Use according to any one of Claims 1 to 13, **characterized in that** the crosslinked acrylamide/neutralized 2-acrylamido-2-methylpropanesulfonic acid copolymer is dispersed at a concentration of 35 to 45% by weight in an oil-in-water emulsion containing 15 to 25% by weight of a mixture of C₁₂-C₁₃ isoparaffin hydrocarbons, 3 to 8% by weight of ether of lauryl alcohol and of polyethylene glycol containing 7 mol of ethylene oxide, and water.

15. Use according to Claim 14, **characterized in that** the oil-in-water emulsion containing the crosslinked acrylamide/neutralized 2-acrylamido-2-methylpropanesulfonic acid copolymer is present in the aqueous dispersion in proportions such that the copolymer concentration is between 0.05 and 10% by weight of active material with respect to the total weight of the dispersion.

16. Cosmetic composition in the form of an aqueous dispersion intended for hair treatment, **characterized in that** the aqueous dispersion is as defined in any one of Claims 1 to 15.

17. Composition according to Claim 16, **characterized in that** it additionally contains adjuvants commonly used in cosmetics, chosen from fragrances, dyes, preserving agents, vegetable, animal or synthetic oils, proteins, conditioning agents, anionic, nonionic, amphoteric or cationic surface-active agents, sequestering agents, foam stabilizing agents, polymers, sunscreening agents, propellants or substances active on the cosmetic level.

18. Composition according to either of Claims 16 or 17, **characterized in that** it has a pH of between 3 and 10 and preferably between 5 and 7.

19. Composition according to any one of Claims 16 to 18, intended for hair treatment, **characterized in that** it is provided in the form of a shampoo, of a rinsing product, to be applied before or after a shampoo, before, during or after dyeing or bleaching, before or after a permanent wave or hair straightening, in an intrapermanent lotion or as non-rinsed styling products.

20. Process for cosmetic hair treatment, **characterized in that** at least one composition as defined in Claim 19 is applied to the hair.

## Patentansprüche

1. Verwendung einer wässrigen Dispersion zur kosmetischen topischen Behandlung der Haare,
**dadurch gekennzeichnet, daß**
die genannte Dispersion, in einem kosmetisch oder physiologisch geeigneten wässrigen Milieu, mindestens ein Organopolysiloxan und ein vernetztes Copolymer aus Acrylamid und neutralisierter 2-Acrylamido-2-methylpropansulfonsäure enthält, mit der Maßgabe, daß das Organopolysiloxan nicht aus linearen Polydimethylsiloxanen einer Viskosität von weniger als 10⁻¹ m²/s ausgewählt ist.

2. Verwendung gemäß Anspruch 1,
**dadurch gekennzeichnet, daß**
das Organosiloxan ein flüchtiges Silicon mit einem Siedepunkt von 60 bis 260°C ist, ausgewählt aus:
(i) zyklischen Siliconen mit 3 bis 7 und vorzugsweise 4 bis 5 Siliziumatomen oder aus Cyclocopolymeren des Typs Dimethylsiloxan/Methylalkylsiloxan,
(ii) linearen Siliconen mit 2 bis 9 Siliziumatomen und einer Viskosität von weniger als oder gleich 5 x 10⁻⁶ m²/s bei 25°C.

3. Verwendung gemäß Anspruch 1 oder 2,
**dadurch gekennzeichnet, daß**
das Organopolysiloxan ein nicht-flüchtiges Silicon ist, ausgewählt aus Polyalkylsiloxanen, Polyalkylarylsiloxanen, Gummi- und Harzprodukten von Silicon, organomodifizierten Polysiloxanen sowie aus deren Mischungen.

4. Verwendung gemäß Anspruch 3,
**dadurch gekennzeichnet, daß**
das Organopolysiloxan ausgewählt ist aus:
A) Poly-C₁₋₂₀-alkylsiloxanen, linearen Polydimethylsiloxanen mit endständigen Trimethylsilylgruppen und linearen Polydimethylsiloxanen mit endständigen Trihydroxysilylgruppen einer Viskosätit von mehr als 10⁻¹ m²_{/s},
B) linearen oder verzweigten Polydimethylphenylsiloxanen oder Polydimethyldiphenylsiloxanen einer Viskosität von 10⁻⁵ bis 5 x 10⁻² m²/s bei 25°C;
C) Gummiprodukten einer Molekularmasse von 200 000 bis 1 000 000, die alleine oder in Form einer Mischung in einem Lösungsmittel eingesetzt werden, ausgewählt aus der Gruppe bestehend aus den folgenden Copolymeren:
- Poly((dimethylsiloxan)/(methylvinylsiloxan)),
- Poly((dimethylsiloxan)/(diphenylsiloxan)),
- Poly((dimethylsiloxan)/(phenylmethylsiloxan)),
- Poly((dimethylsiloxan)/(diphenylsiloxan)/(methyl vinylsiloxan));
sowie aus den folgenden Mischungen:
- aus Mischungen, die aus einem am Kettenende hydroxylierten Polydimethylsiloxan und einem zyklischen Polydimethylsiloxan gebildet sind;
- aus Mischungen, die aus einem Polydimethylsiloxan-Gummi und einem zyklischen Silicon gebildet sind;
- aus Mischungen aus zwei Polydimethylsiloxanen unterschiedlicher Viskositäten;
D) Harzen von Organopolysiloxanen, enthaltend die Einheiten R₂SiO_{2/2}, RSiO_{3/2} und SiO_{4/2}, in denen R eine Kohlenwasserstoffgruppe mit 1 bis 6 Kohlenstoffatomen oder eine Phenylgruppe darstellt.

5. Verwendung gemäß Anspruch 3,
**dadurch gekennzeichnet, daß**
das Organopolysiloxan in seiner allgemeinen Struktur eine oder mehrere organofunktionelle Gruppen aufweist, die direkt an die Siloxankette oder über das Zwischenglied eines Kohlenwasserstoffrestes gebunden sind, und daß es aus Polysiloxanen ausgewählt ist, die enthalten:
a) Polyethylenoxi- und/oder Polypropylenoxigruppen;
b) perfluorierte Gruppen;
c) Hydroxyacylaminogruppen;
d) Thiolgruppen;
e) Amingruppen, die substituiert sind oder nicht;
f) Carboxylatgruppen;
g) Hydroxyalkylgruppen der Formel: 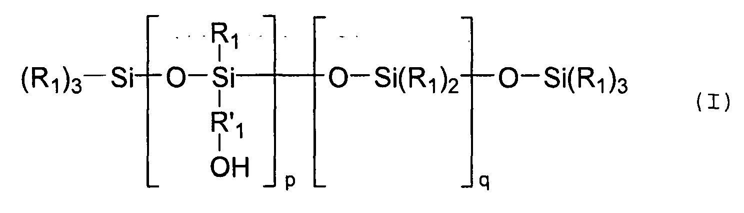 worin gilt:
- die Reste R₁ sind, gleich oder verschieden, aus Methyl- und Phenylresten ausgewählt, wobei mindestens 60 Mol% der Reste R₁ Methylreste sind;
- der Rest R'₁ ist eine zweiwertige C₂₋₁₈-Alkylenkohlenwasserstoffkette;
- p beträgt 1 bis 30;
- q beträgt 1 bis 150;
h) Alkoxygruppen;;
i) Acyloxialkylgruppen der folgenden Formel: 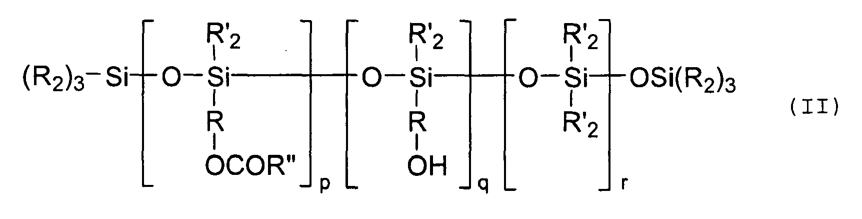 worin gilt:
- R₂ bedeutet einen Methyl-, Phenyl-, OCOR''- oder Hydroxylrest, wobei nur einer der Reste R₂ pro Siliziumatom ein OH-Rest ist;
- R'₂ bedeutet einen Methyl- oder Phenylrest, wobei mindestens 60 Mol% aller Reste R₂ und R'₂ durch den Methylrest dargestellt sind;
- R" bedeutet einen C₈₋₂₀-Alkyl- oder -Alkenylrest;
- R bedeutet einen linearen oder verzweigten C₂₋₁₈-Alkylenkohlenwasserstoffrest;
- r beträgt 1 bis 120;
- p beträgt 1 bis 30;
- q beträgt 0 oder weniger als 0,5 p, wobei p+q 1 bis 30 beträgt,
wobei die Polyorganosiloxane der Formel (II)
CH₃-Si-O_{2/2}-OH-Gruppen enthalten können,
und zwar in Mengenanteilen, die 15 % der Summe p+q+r nicht übersteigen;
j) quaternäre Ammoniumgruppen;
k) amphotere oder betainische Gruppen;
l) Bisulfit-Gruppen.

6. Verwendung gemäß einem jeden der Ansprüche 1 bis 5,
**dadurch gekennzeichnet, daß**
das Organopolysiloxan ausgewählt ist aus:
1) nicht-flüchtigen Siliconen des Typs linearer Polyalkylsiloxane mit endständigen Trimethylsilylgruppen oder des Typs Polyalkylphenylsiloxan;
2) Mischungen aus Organosiloxanen und zyklischen Siliconen;
3) Fluorsiliconen des Typs Polyalkylsiloxan mit endständigen Trimethylsilylgruppen, wobei die Kette mit Trifluorpropylgruppen substituiert ist.

7. Verwendung gemäß jedem der Ansprüche 1 bis 6,
**dadurch gekennzeichnet, daß**
das Copolymer aus Acrylamid/2-Acrylamido-2-methylpropansulfonsäure durch ein Vernetzungsmittel mit olefinischer Mehrfachungestättigtheit, ausgewählt aus Tetraallyloxyethan, Allylsucrose, Allylpentaerythrit und Methylenbisacrylamid, vernetzt und teilweise oder ganz mit Soda, Pottasche, Ammoniak oder einem Amin neutralisiert ist.

8. Verwendung gemäß Anspruch 7,
**dadurch gekennzeichnet, daß**
das Copolymer durch Copolymerisation von 70 bis 55 Mol% Acrylamid und 30 bis 45 Mol% Natrium-2-acrylamido-2-methylpropansulfonat in Gegenwart von 10⁻⁴ bis 4 x 10⁻⁴ Mol Vernetzungsmittel pro Mol der Mischung der beiden Monomeren erhältlich ist.

9. Verwendung gemäß jedem der Ansprüche 1 bis 8,
**dadurch gekennzeichnet, daß**
das Organopolysiloxan in Mengenanteilen von 0,5 bis 50 Gew.%, bezogen auf das Gesamtgewicht der Dispersion, und das Copolymer in Mengenanteilen von 0,05 bis 10 Gew.% vorhanden sind.

10. Verwendung gemäß jedem der Ansprüche 1 bis 9,
**dadurch gekennzeichnet, daß**
die wässrige Dispersion enthält:
a) Organopolysiloxan;
b) vernetztes Copolymer aus Acrylamid und neutralisierter 2-Acrylamido-2-methylpropansulfonsäure;
c) einen nicht-ionischen Emulgator;
d) einen Kohlenwasserstoff oder eine Mischung aus isoparaffinischen Kohlenwasserstoffen mit erhöhtem Siedepunkt.

11. Verwendung gemäß Anspruch 10,
**dadurch gekennzeichnet, daß**
der Emulgator aus Sorbitanfettsäureestern, Fettsäureestern, die ethoxyliert sind oder nicht, Fettalkoholen, die ethoxyliert sind oder nicht, aus Copolymeren des Typs mit Ethylenoxid/Propylenoxid- oder Ethylenoxid/Butylenoxid-Sequenzen oder aus deren Mischungen ausgewählt ist.

12. Verwendung gemäß Anspruch 10 oder 11,
**dadurch gekennzeichnet, daß**
der Emulgator in Mengenanteilen von 0,01 bis 1,5 Gew.% und der oder die isoparaffinischen Kohlenwasserstoffe in Mengenanteilen von 0,02 bis 6,5 Gew.% vorhanden sind, bezogen auf das Gesamtgewicht der Dispersion.

13. Verwendung gemäß jedem der Ansprüche 10 bis 12,
**dadurch gekennzeichnet, daß**
der nicht-ionische Emulgator der Laurylether von 7 Mol Ethylenoxid enthaltendem Polyethylenglycol ist und daß man eine Mischung aus isoparaffinischen C₁₂₋₁₃-Kohlenwasserstoffen verwendet.

14. Verwendung gemäß jedem der Ansprüche 1 bis 13,
**dadurch gekennzeichnet, daß**
das vernetzte Copolymer aus Acrylamid und neutralisierter 2-Acrylamido-2-methylpropansulfonsäure in einer Menge von 35 bis 45 Gew.% in einer Öl-in-Wasser-Emulsion dispergiert ist, die 15 bis 25 Gew.% einer Mischung aus isoparaffinischen C₁₂₋₁₃-Kohlenwasserstoffen, 3 bis 8 Gew.% Laurylether von 7 Mol Ethylenoxid enthaltendem Polyethylenglycol und Wasser enthält.

15. Verwendung gemäß Anspruch 14,
**dadurch gekennzeichnet, daß**
die Öl-in-Wasser-Emulsion, die das vernetzte Copolymer aus Acrylamid und neutralisierter 2-Acrylamido-2-methylpropansulfonsäure enthält, in der wässrigen Dispersion in solchen Mengenanteilen vorhanden ist, daß die Konzentration an Copolymer 0,05 bis 10 Gew.% Aktivmasse ausmacht, bezogen auf das Gesamtgewicht der Dispersion.

16. Kosmetische Zusammensetzung in Form einer wässrigen Dispersion zur Behandlung der Haare,
**dadurch gekennzeichnet, daß**
die wässrige Dispersion so beschaffen ist, wie sie in jedem der Ansprüche 1 bis 15 definiert ist.

17. Zusammensetzung gemäß Anspruch 16,
**dadurch gekennzeichnet, daß**
sie ausserdem in der Kosmetik gewöhnlich verwendete Hilfsstoffe enthält, ausgewählt aus Parfüm-Produkten, Färbemitteln, Konservierungsstoffen, pflanzlichen, tierischen oder synthetischen Ölen, Proteinen, Konditioniermitteln, anionischen, nicht-ionischen, amphoteren oder kationischen oberflächenaktiven Mitteln, Sequestriermitteln, Schaumstabilisatoren, Polymeren, Sonnenfiltermitteln, Treibmitteln und aus Substanzen, die auf dem kosmetischen Plan wirksam sind.

18. Zusammensetzung gemäß jedem der Ansprüche 16 oder 17,
**dadurch gekennzeichnet, daß**
sie einen pH von 3 bis 10 und vorzugsweise von 5 bis 7 aufweist.

19. Zusammensetzung gemäß jedem der Ansprüche 16 bis 18 zur Behandlung der Haare,
**dadurch gekennzeichnet, daß**
sie in Form eines Schamponierprodukts, eines Produkts zur Spülung, zur Aufbringung vor oder nach einem Schamponiervorgang, vor, bei oder nach einer Färbung oder Entfärbung, vor oder nach einer Dauerwelle oder einem Entfrisiervorgang, als Lotion zur Aufbringung zwischen den Stufen einer Dauerwelle oder als nicht zur Spülung vorgesehene Produkte zum Frisieren vorliegt.

20. Verfahren zur kosmetischen Behandlung der Haare,
**dadurch gekennzeichnet, daß**
man auf diese mindestens eine im Anspruch 19 definierte Zusammensetzung aufbringt.
